# EUROPÄISCHE PATENTSCHRIFT

(11) **EP 0 513 133 B1**
(45) Veröffentlichungstag und Bekanntmachung des Hinweises auf die Patenterteilung: **17.11.1994**
(21) Anmeldenummer: 91903639.2
(22) Anmeldetag: 25.01.1991
(51) Int. Cl.: C12N 9/00, C12N 15/82, C12N 15/54, C12N 5/04, C12N 1/00, A01H 1/06

(54) **EXPRESSION EINER MULTIGEN RNA MIT SELF-SPLICING AKTIVITÄT**
EXPRESSION OF A MULTIGENE RNA WITH SELF-SPLICING ACTIVITY
EXPRESSION D'UN ARN MULTIGENES A ACTIVITE D'AUTO-CLIVAGE-RACCORDEMENT

(30) Priorität: 01.02.1990 DE 4002885
(43) Veröffentlichungstag der Anmeldung: 19.11.1992
(73) Patentinhaber: Hoechst Schering AgrEvo GmbH, 13342 Berlin (DE)
(72) Erfinder: MÜLLNER, Hubert, D-6233 Kelkheim (DE); SCHNEIDER, Rudolf, D-6233 Kelkheim (DE); UHLMANN, Eugen, D-6246 Glashütten (DE)
(86) Internationale Anmeldenummer: EP9100145
(87) Internationale Veröffentlichungsnummer: WO9111511

(56) Entgegenhaltungen:
- EP-A- 0 321 201
- EP-A- 0 421 376
- Scientific American, vol. 255, no. 5, November 1986 (New York, US) T.R. CEch."RNA as an enzyme", pages 76-84 see the whole article
- Nature, vol. 334, 18 August 1988, J. Haseloff et al.: "Simple RNA enzymes with new and highly specific endoribonuclease activities", pages 585-591 see the whole article.

## Beschreibung

RNA-Moleküle können unter geeigneten Voraussetzungen ohne Beteiligung von Proteinen an anderen RNA-Molekülen Reaktionen katalysieren oder autokatalytisch Fragmente aus eigenen Molekülen abspalten. So wird vom 3'-Ende der 23s rRNA von Tetrahymena thermophila autokatalytisch ein Intron mit 413 Nukleotiden entfernt und in eine zirkuläre Form übergeführt. Dies geschieht durch eine Reihe von Phosphoestertransfer-Reaktionen mit Beteiligung von Guanosin-Kofaktoren (Cech, T.R., Nature 30, 578-583 (1983)). Je nach RNA Substrat oder den gewählten Reaktionsbedingungen kann das Intron als spezifische Ribonuclease, terminale Transferase, Phosphotransferase oder saure Phosphatase funktionieren. Dabei kann ein RNA-Molekül einige Umsetzungen vornehmen, ohne selbst verändert zu werden und verhält sich in dieser Hinsicht wie ein Enzym. Für RNA-Moleküle mit diesen Eigenschaften wurde deshalb der Begriff Ribozym geprägt.

Ähnliche Reaktionen ohne Beteiligung von Proteinen konnten auch für einige viroide RNAs und Satelliten-RNAs gezeigt werden. So scheint für Avocado Sunblotch Viroid (ASBV) (Hutchins, C.J. et al. Nucleic Acids Res. 14, 3627-3640 (1986)), Satelliten-RNA von Tobacco Ringspot Virus (sTobRV) (Prody, G.A. et al., Science 231, 1577-1580 (1986)) und Satelliten-RNA von Luzerne Transient Streak Virus (sLTSV) (Forster A.C. et al., Cell 49, 211-220 (1987)) eine Selbst-Prozessierung eine essentielle Reaktion für die Vermehrung zu sein. Während der Replikation dieser RNAs werden vermutlich zirkuläre Formen gebildet, die als "Templates" zur Synthese von RNAs mit Überlänge führen. Diese Transkripte werden durch die selbstkatalysierten, endonucleolytischen Reaktionen auf die richtige Genomlänge zurechtgeschnitten.

Die Strukturen der RNAs, die diese vermutlich für die Reaktion einnehmen, wurden als "hammerheads" beschrieben (Forster A.C. et al., Cell 49, 211-220 (1987)); Haseloff, J. et al., Nature 334, 585-591 (1988)).

Die Schnittstellen für diese RNA-Enzyme sind spezifisch und müssen bestimmte strukturelle Voraussetzungen aufweisen, damit eine Prozessierung erfolgen kann.

Es wurde nun überraschend gefunden, daß Wirtszellen beliebiger Organismen mit Vektoren, die DNA kodierend für Ribozym RNA verknüpft mit funktionellen Genen enthalten, transformiert werden können, so daß die genannte RNA exprimiert und anschließend gespleißt wird.

Die Erfindung betrifft somit:
1. Ein Hybridgen, bestehend aus einer Gensequenz, kodierend für Ribozym-RNA, und einem oder unterschiedlichen funktionellen Genen, in einer oder mehreren Kopien, wobei die Gensequenzen über einen Spacer, der auf der RNA-Ebene ein Substrat für das Ribozym darstellt, verknüpft sind.
2. Wirtszellen, die das unter 1. charakterisierte Gen enthalten.

Im folgenden wird die Erfindung detailliert beschrieben, insbesondere in ihren bevorzugten Ausführungsformen. Ferner wird die Erfindung durch den Inhalt der Ansprüche definiert.

Unter einem funktionellen Gen, versteht man einen DNA-Abschnitt im Genom, der für ein Polypeptid codiert. Das Polypeptid kann für sich ein funktionelles Protein darstellen oder als Untereinheit eines Enzymkomplexes fungieren.

Das erfindungsgemäße Gen ist so konstruiert, daß von einem Promotor aus mehrere Gene, beispielsweise ganze Synthesewege für die Herstellung von z.B. Aminosäuren, wie Glycin, Nukleotiden, Sekundär Metaboliten wie Antibiotika, Cofaktoren von Enzymen, Hormonen, wie das Schilddrüsenhormon, in Pflanzen oder Mikroorganismen exprimiert werden können. Es sollen damit einmal artfremde Stoffe in entsprechenden Pflanzen oder Mikroorganismen hergestellt werden bzw. soll die Ausbeute in Pflanzen oder Mikroorganismen, die diese Stoffe natürlich synthetisieren, erhöht werden. Gene kodierend für entsprechende Polypeptide können unter Verwendung von Kontrollsequenzen erfindungsgemäß Anwendung finden.

Ferner ist es möglich, ein Gen für ein bestimmtes funktionelles Protein in mehreren Kopien einzusetzen, z.B. um die Ausbeute eines Proteins wie Insulin oder Transaminase zu erhöhen.

Weiterhin ist die Expression eines oder mehrerer Selektionsmarker mit dem erfindungsgemäßen System möglich. Dazu können beispielsweise Gene für folgende Proteine Anwendung finden: β-Lactamase, β-Galaktosidase, Phosphinotricin-acetyl-transferase, Chloramphenicol-acetyl-transferase oder Thymidinkinase.

Bevorzugt wird mit dem Acetyltransferasegen für das Herbizid Phosphinotricin und dem Cat-Gen aus dem Transposon Tn9 für die Chloramphenicolacetyltransferase gearbeitet.

Das Acetyltransferasegen aus Streptomyces viridochromogenes (Wohleben, W. et al., Gene 70, 25-37 (1988)), kann aus synthetisch hergestellten Oligonukleotiden zusammengesetzt werden, wobei es für die Expression in Pflanzen auch modifiziert werden kann. Das Gen vermittelt Resistenz gegen Phosphinotricin in transgenen Pflanzen, die das Produkt konstitutiv exprimieren. Das Cat-Gen bewirkt Resistenz gegen Chloramphenicol. Ebenso wie das Acetyltransferasegen acetyliert das Cat-Gen sein Produkt. Das Cat-Gen stammt aus Tn9 (Alton, N.K. et al., Nature 15 282, 864-866, 1979), ist aber auch käuflich erhältlich.

Die Gene sind durch Ribozymsubstratsequenzen, sogenannte Spacer verknüpft und werden durch eine Ribozymstrukturdomäne desselben Moleküls freigesetzt. Auf diese Weise können mindestens 2 bis zu ca. 25 oder mehr Gensequenzen in einem Organismus exprimiert werden. Die Freisetzung kann auch über ein getrennt exprimiertes Ribozymmolekül erfolgen.

Die entsprechend transkribierte RNA ist im wesentlichen so zusammengesetzt, daß sich die Ribozyme bevorzugt am 3'- bzw. 5'-Ende des RNA Moleküls befinden. Als Spacer wird eine Sequenz von 40-50 Nukleotiden eingeschoben, die im ganzen oder in einem mindestens 10, bevorzugt 15-35 Nukleotide zählenden Teilbereich zur Sequenz des Ribozyms komplementär ist. Die Ribozym-Sequenz der RNA kann sich so an den Spacer anlagern und diesen unmittelbar hinter einer definierten Sequenz schneiden. Als Ribozymschnittstelle wird bevorzugt ein GUC-Triplet verwendet. Die Anzahl der verknüpften Gene kann jeweils durch Einfügen von weiteren Spacern vermehrt werden, wobei deren Sequenz gleich oder verschieden vom ersten eingefügten Spacer sein kann. Falls sie verschieden ist, wird im gleichen RNA-Molekül eine weitere Ribozymstrukturdomäne passend zu dieser Sequenz geschaffen.

Die für diese RNA benötigten Spacer- und Ribozym-Sequenzen können synthetisch hergestellt werden. Die Verknüpfung mit den Genen erfolgt über geeignete ansynthetisierte Linker.

Spacer und Ribozym werden analog zu Ribozymstrukturen der Natur synthetisiert (Uhlenbeck, O.C., Nature 328, 596-600, 1987). Dabei können diese Sequenzen natürlich vorkommende Ribozyme nachbilden (Forster A.C. et al., Cell 49, 211-220, (1987)) oder so gestaltet werden, daß die essentiellen Strukturen des Ribozyms vorhanden sind, für nicht essentielle Teile aber andere Sequenzen gewählt werden. Bei der Grundkonstruktion kann im wesentlichen nach Haseloff, J. et al., Nature 334, 585-591, 1988 vorgegangen werden. In den Spacerteil der RNA wird eine GUC-Sequenz eingebaut, um die in 5'- und 3'-Richtung Sequenzen liegen, die komplementär zu einer beschriebenen Ribozym-Sequenz sind.

Spacer und Ribozym auf der RNA-Ebene können im Schema wie folgt aussehen: 
5-NNNNNNNNNNNNNGUC NNNNNNNNNNNNNN-3 ← Subatrat-RNA
3-KKKKKKKKCA KKKKKKK-5
A C
A U
G G
A A
C G G V
V V
V V
VL VL VL
VL VL VL
Ribozym
wobei
- N: Nukleotide der Substrat-RNA, A, C, G oder U
- K: komplementäre Nukleotide zu N im Ribozym
- V: variable Nuklectide A, C, G oder U im Ribozym und
- V_{L}: variable Nukleotide A, C, G oder U im Loop des Ribozyms bedeutet.

Die Nukleotidanzahl von V_{L} kann 0-550 betragen.

Das erfindungsgemäße Gen wird in einen intermediären Vektor mit Pflanzen-Promotor kloniert. Derartige Vektoren sind beispielsweise die Plasmide pNCN (Fromm M. et al., PNAS 82, 5824-5826 (1985) oder pNOS (An G. et al. EMBO J. 4, 277-276 (1985) oder bevorzugt pDH51 (Pietrzak, M. et al. NAR 14, 5857-5861, (1986).

Nach anschließender Transformation von E. coli, wie z.B. E. coli MC 1061, DH1, DK1, GM48 oder XL-1, werden positive Klone nach an sich bekannten Methoden (Maniatis et al., Lab. Manual), wie Plasmidminipräparation und Spaltung mit einem entsprechenden Restriktionsenzym, identifiziert. Diese positiven Klone werden dann in einen binären Pflanzenvektor subkloniert. Als Pflanzenvektoren können pGV3850 (Zambrysky, P. et al., EMBO J. 2, 2143-2150 (1983)) oder pOCA18 (Olszewski, N., NAR 16, 10765-10782, (1988)) eingesetzt werden. Vorteilhaft wird mit pOCA18 gearbeitet.

Die erhaltenen binären Pflanzenvektoren, die einen Pflanzenpromotor mit dem angehängten DNA-Fragment, das wie oben angegeben konstruiert ist, in der T-DNA enthalten, werden verwendet, um Pflanzen zu transformieren. Dies kann durch Techniken wie Elektroporation oder Mikroinjektion erfolgen.

Bevorzugt wird die Kokultivierung von Protoplasten oder die Transformation von Blattstückchen mit Agrobakterien angewandt. Dazu wird das Pflanzenvektorkonstrukt durch Transformation mit gereinigter DNA oder, vermittelt über einen Helferstamm wie E. coli SM10 (Simon R. et al., Biotechnology 1, 784-791 (1983)), in Agrobakterium tumefaciens wie A 282 mit einem Ti Plasmid über ein "triparental mating" transferiert. Direkte Transformation und triparental mating wurden, wie in, "Plant Molecular Biology Manual" (Kluwer Academic Pubishers, Dardrecht (1988)) beschrieben, durchgeführt.

Es können grundsätzlich alle Pflanzen mit den die erfindungsgemäße, konstruierte DNA tragenden binären Pflanzenvektoren transformiert werden. Bevorzugt sind dikotyledone Pflanzen, insbesondere Nutzpflanzen, die beispielsweise Stärke, Kohlenhydrate, Eiweiße oder Fette in nutzbaren Mengen in ihren Organen produzieren oder speichern oder die Obst und Gemüse produzieren oder die Gewürze, Fasern und technisch verwertbare Produkte oder Arzneimittel, Farbstoffe oder Wachse liefern ebenso wie Futterpflanzen. Als Beispiel sollen Tomate, Erdbeere, Avocado, sowie Pflanzen, die tropische Früchte tragen, z.B. Papaya, Mango, aber auch Birne, Apfel, Nektarine, Aprikose oder Pfirsich genannt werden. Ferner sollen als zu transformierende Pflanzen beispielhaft alle Getreidearten, Raps, Kartoffeln, Sojabohne, Baumwolle, Mais, Zuckerrübe oder Sonnenblume, aufgeführt werden.

Die transformierten Zellen werden mit Hilfe eines Selektionsmediums selektiert, zu einem Kallus herangezüchtet und auf einem entsprechenden Medium zur Pflanze regeneriert (Shain et al., Theor. appl. Genet. 72, 770-770 (1986); Masson, J. et al., Plant Science 53, 167-176 (1987), Zhan et al., Plant Mol. Biol. 11, 551-559 (1988); McGranaham et al., Bio/Technology 6, 800-804 (1988) Novrate et al., Bio/Technology 7, 154-159 (1989).

Die resultierende Pflanze wird durch die Transformation insofern verändert, als in den Zellen die mit Hilfe der konstruierten Oligonukleotide exprimierte RNA durch die Ribozymaktivität an GUC-Schnittstellen aufgespalten wird, um die Gene freizusetzen.

Eine Anwendung des beschriebenen Systems ist auch in Bakterien, Zellkulturen, Hefen oder anderen eukaryontischen Organismen möglich.

In den folgenden Beispielen wird die Erfindung weitergehend erläutert.

### Beispiele

### 1) Verwendete DNA Strukturen

a) Acetyltransferasegen mit SalI-Linkern 
9 18 27 36 45
5' GTC GAC ATG TCT CCG GAG AGG AGA CCA GTT GAG ATT AGG CCA GCT
3' G TAC AGA GGC CTC TCC TCT GGT CAA CTC TAA TCC GGT CGA
54 63 72 81 90
ACA GCA GCT GAT ATG GCC GCG GTT TGT GAT ATC GTT AAC CAT TAC
TGT CGT CGA CTA TAC CGG CGC CAA ACA CTA TAG CAA TTG GTA ATG
99 108 117 126 135
ATT GAG ACG TCT ACA GTG AAC TTT AGG ACA GAG CCA CAA ACA CCA
TAA CTC TGC AGA TGT CAC TTG AAA TCC TGT CTC GGT GTT TGT GGT
144 153 162 171 180
CAA GAG TGG ATT GAT GAT CTA GAG AGG TTG CAA GAT AGA TAC CCT
GTT CTC ACC TAA CTA CTA GAT CTC TCC AAC GTT CTA TCT ATG GGA
189 198 207 216 225
TGG TTG GTT GCT GAG GTT GAG GGT GTT GTG GCT GGT ATT GCT TAC
ACC AAC CAA CGA CTC CAA CTC CCA CAA CAC CGA CCA TAA CGA ATG
234 243 252 261 270
GCT GGG CCC TGG AAG GCT AGG AAC GCT TAC GAT TGG ACA GTT GAG
CGA CCC GGG ACC TTC CGA TCC TTG CGA ATG CTA ACC TGT CAA CTC
279 288 297 306 315
AGT ACT GTT TAC GTG TCA CAT AGG CAT CAA AGG TTG GGC CTA GGA
TCA TGA CAA ATG CAC AGT GTA TCC GTA GTT TCC AAC CCG GAT CCT
324 333 342 351 360
TCC ACA TTG TAC ACA CAT TTG CTT AAG TCT ATG GAG GCG CAA GGT
AGG TGT AAC ATG TGT GTA AAC GAA TTC AGA TAC CTC CGC GTT CCA
369 378 387 396 405
TTT AAG TCT GTG GTT GCT GTT ATA GGC CTT CCA AAC GAT CCA TCT
AAA TTC AGA CAC CAA CGA CAA TAT CCG GAA GGT TTG CTA GGT AGA
414 423 432 441 450
GTT AGG TTG CAT GAG GCT TTG GGA TAC ACA GCC CGG GGT ACA TTG
CAA TCC AAC GTA CTC CGA AAC CCT ATG TGT CGG GCC CCA TGT AAC
459 468 477 486 495
CGC GCA GCT GGA TAC AAG CAT GGT GGA TGG CAT GAT GTT GGT TTT
GCG CGT CGA CCT ATG TTC GTA CCA CCT ACC GTA CTA CAA CCA AAA
504 513 522 531 540
TGG CAA AGG GAT TTT GAG TTG CCA GCT CCT CCA AGG CCA GTT AGG
ACC GTT TCC CTA AAA CTC AAC GGT CGA GGA GGT TCC GGT CAA TCC
549 558
CCA GTT ACC CAG ATC TGA G· 3'
GGT CAA TGG GTC TAG ACT CAG CTG 5'
b) Spacer mit SalI und HindIII-Linkern 
9 18 27 36
5' TCG ACT TAC GGC TAA AAT GGT CAG TAT CCC CCA AAG GCG GCC GC 3'
3' GA ATG CCG ATT TTA CCA GTC ATA GGG GGT TTC CGC CGG CGT

CGA 5´
c) Cat-Gen aus Tn9 nach Alton et al. Nature 282, 864-866 (1979)
d) Ribozymstrukturdomäne mit HindIII- und PstI-Linkern

9 18 27 36 45
5' AGC TGC GGC CGC TTA CGG CTA AAA TGG TCA GTA TCC CCC AAA GGG
3' CG CCG GCG AAT GCC GAT TTT ACC AGT CAT AGG GGG .TTT CCC
54 63 72 81 90
GTA CCC CTT TCG GGC ATA CTC TGA TGA GTC CGT GAG GAC GAA ACC
CAT GGG GAA AGC CCG TAT GAG ACT ACT CAG GCA CTC CTG CTT TGG
99 108
ATT TTA GCC GTA ACT GCA 3'
TAA AAT CGG CAT TG 5' .

Die Oligonukleotide unter a), b) und d) wurden nach der Phosphoramidit Methode mit einem DNA-Synthesizer synthetisiert.

### 2) Klonierung der Fragmente

Die unter 1a)-d) bezeichnete DNA wurde in gleichen molaren Mengen ligiert und in die SalI/PstI Stellen des Vektros pDH51 eingebaut. Positive Klone wurden durch Hybridisierung mit allen 4 verwendeten radioaktiv markierten DNA-Abschnitten identifiziert.

### 3) Klonierung in pOCA18

Das Plasmid pOCA18 wird in Olszewski, N. et al. NAR 16, 10765-10782 (1988) nacharbeitbar beschrieben.

Ein 2,4 kbp langes NosI/HindIII Fragment wurde aus dem beschriebenen Vektor pDH51 mit der inserierten Konstruktion isoliert und nach Auffüllen der Enden in einen Bam HI geschnittenen und aufgefüllten pOCA18 Vektor kloniert. Positive Klone wurden durch Hybridisierung mit ³²P markierter DNA nachgewiesen.

### 4) Transformation von Agrobakterien

Der pOCA18 Vektor mit dem beschriebenen 35S Promotor/Insert wurde in den Agrobakterienstamm A 282 (Pharmacia Freiburg, BR Deutschland, oder ATCC 37349, USA) überführt. Dies geschah durch ein Triparental Mating mit Hilfe des E. coli Stammes SM10 (Simon, R. et al. Bio/Technology 1, 784-791, 1983). Dazu gab man gleiche Mengen der Bakterien gemeinsam über Nacht auf einen Filter, spülte mit 2 ml 10 mM MgSO₄ ab und gab Aliquots davon auf YEB-Platten mit Tetrazyklin und Rifampicin (YEB: 1 % Hefeextrakt, 1 % Pepton, 0,5 % NaCl). Positive Agrobakterien konnten durch Hybridisierung nachgewiesen werden.

### 5) Transformation von Tabak

Die Agrobakterien wurden in YEB Medium (1 % Hefeextrakt, 1 % Pepton, 0,5 % NaCl) mit Tetrazyklin und Rifampicin angezüchtet. 20 ml der Bakterien wurden abzentrifugiert, einmal in YEB Medium gewaschen und in 20 ml 10 mM MgSO₄ suspendiert in eine Petrischale gegeben. Als Pflanzenmaterial wurde Nicotiana tabacum Wisconsin 38 verwendet. Die Pflanzen waren 4 Wochen unter sterilen Bedingungen auf 2MS Medium Murashige T. et al., Physiol. Plant 15, 473-497 (1962) bei 25°C mit 16 Std. Licht pro Tag kultiviert worden. Von diesen Pflanzen wurde ein 1 cm² Blattstückchen abgeschnitten, mit sterilem Schmirgelpapier verwundet und 30 sec in die Bakterienkultur eingetaucht. Die Blattstückchen wurden 2 Tage bei 25°C auf MS Medium, wie oben für 2MS beschrieben, gehalten und anschließend mit flüssigem 2MS Medium gewaschen. Danach kamen die Blattstückchen auf MSC 10 Platten (wie MS mit 1,5 % Agar) mit 100 µg/ml Kanamycin. Nach 5-6 Wochen konnten regenerierte Pflanzen in größere Gefäße umgepflanzt werden, wo sie nach 2-3 Wochen Wurzeln bildeten.

### 6) Nachweis der Transformation

Aus etwa 8 Wochen alten transformierten Tabakpflanzen wurde mit Standardmethoden (Maniatis et al., Lab. Journal) DNA isoliert, auf Nitrozellulosemembranen transferiert und mit ³²P markierter Insert-DNA hybridisiert.

In der DNA der Pflanze konnten ein Einbau der gewünschten Sequenzen nachgewiesen werden.

### 7) Nachweis der Expression der RNA

Aus den eben erwähnten Tabakpflanzen wurde aus einer zweiten Blattprobe RNA isoliert, aus einem Formaldehydgel auf Nitrozellulose transferiert und wie oben hybridisiert. Es konnten verschiedene Banden nachgewiesen werden, die die erwarteten Größen zeigten.

### 8) Nachweis der in vitro Funktion der Ribozym RNA

Aus den pBluescript SK+ Klonen mit dem insertierten Gesamt-Oligo wurde mit T3 bzw. T7 Polymerase in einem Reaktionsansatz (Stratagene, Product Information zu SK+) die multifunktionelle RNA produziert und anschließend isoliert. Hybridisierung dieser RNA mit einzelnen Komponenten zeigte eine Aufspaltung der RNA.

### 9) Nachweis der in vivo Aktivität der Gene

Transformierte Pflanzen zeigten Wachstum auf 2MS Medium mit Phosphinotricin. Im Sprühversuch erwiesen sich die Pflanzen gleichfalls als resistent. Ein Versuch zur Acetylierung von Chloramphenicol zeigte, daß die Pflanzen ein aktives Enzym exprimieren.

## Patentansprüche

1. Hybridgen, bestehend aus einer Gensequenz, kodierend für Ribozym-RNA, und einem oder unterschiedlichen funktionellen Genen in einer oder mehreren Kopien, wobei die Gensequenzen jeweils über einen Spacer, der auf der RNA-Ebene ein Substrat für das Ribozym darstellt, verknüpft sind.

2. Hybridgen nach Anspruch 1, dadurch gekennzeichnet, daß das funktionelle Gen für die Phosphinothricin-Acetyltransferase und/oder für die Chloramphenicol-Acetyltransferase kodiert.

3. Wirtszelle, enthaltend ein Gen nach Anspruch 1 oder 2.

4. Mikroorganismus oder Pflanzen, Pflanzenzellen, sowie Teile oder Samen der Pflanzen, enthaltend ein Gen nach Anspruch 1 oder 2.

## Claims

1. A hybrid gene composed one or more copies of a gene sequence coding for ribozyme RNA and of one or various functional genes, in one or more copies, the gene sequences being linked in each case via a spacer which, on the RNA level, represents a substrate for the ribozyme.

2. A hybrid gene as claimed in claim 1, wherein the functional gene codes for phosphinothricin acetyltransferase and/or for chloramphenicol acetyltransferase.

3. A host cell containing a gene as claimed in claim 1 or 2.

4. A microorganism or a plant, plant cell, and a part or a seed of the plant, containing a gene as claimed in claim 1 or 2.

## Revendications

1. Gène hybride, composé d'une séquence de gènes, codant pour l'ARN ribozymique, et d'un ou de plusieurs gènes fonctionnels différents, en une ou plusieurs copies, les séquences de gènes étant chaque fois liées par l'intermédiaire d'un segment espaceur qui représente sur l'ARN substrat un substrat pour le ribozyme.

2. Gène hybride selon la revendication 1, caractérisé en ce que le gène fonctionnel code pour la phosphinotricine-acétyltransférase et/ou pour la chloramphénicol-acétyltransférase.

3. Cellule hôte contenant un gène selon la revendication 1 ou 2.

4. Microorganisme ou plantes, cellules végétales ainsi que parties ou graines de plantes contenant un gène selon la revendication 1 ou 2.
